Europäisches Patentamt

European Patent Office

Office européen des brevets

(11) Publication number: **0 279 616**
**A2**

# (12) EUROPEAN PATENT APPLICATION

(21) Application number: **88301247.8**

(22) Date of filing: **15.02.88**

(51) Int. Cl.⁴: **A 61 K 37/36**
**A 23 K 1/165**

(30) Priority: **17.02.87 US 14876**

(43) Date of publication of application:
**24.08.88 Bulletin 88/34**

(84) Designated Contracting States:
**AT BE CH DE ES FR GB GR IT LI LU NL SE**

(71) Applicant: **INTERNATIONAL MINERALS AND CHEMICAL CORPORATION**
P.O. Box 207 1401 South Third Street
Terre Haute Indiana 47808 (US)

(72) Inventor: **Ivy, Richard E.**
R.R. 22 Box 473
Terre Haute Indiana 47802 (US)

**Mouzin, Douglas E.**
1980 W. 76th Drive
Terre Haute Indiana 47802 (US)

(74) Representative: **Holmes, Michael John et al**
Frank B. Dehn & Co. European Patent Attorneys Imperial
House 15-19 Kingsway
London, WC2B 6UZ, (GB)

(54) **Swine growth promotion.**

(57) Bovine somatotropin is used to promote growth, improve weight gain, and increase feed utilization efficiency in swine.

EP 0 279 616 A2

## Description

## SWINE GROWTH PROMOTION

This invention relates generally to methods for promoting growth in swine using somatotropin and particularly to a method for using bovine somatotropin to promote growth, improve weight gain, and increase feed utilization efficiency in swine.

Background of the Invention

Somatotropin (ST), sometimes referred to as Growth Hormone (GH) in the art, is normally produced by the pituitary throughout an animal's life, although apparently in higher amounts during the pre-adult period. ST is known to promote skeletal growth, nitrogen retention, protein synthesis and to affect glucose and lipid metabolism. Accordingly, ST is recognized as a general anabolic agent.

Although the mechanism of ST activity is not well understood, it has been demonstrated that the administration of exogeneous ST of the same species as the test subject markedly increases the rate of growth, weight gain, and meat production in animals. For example, in one test, the average rate of weight gain in pigs receiving daily injections of purified porcine ST was 2.26 pounds per day (as compared to an average weight gain of 2.19 lbs/day in control pigs). More importantly, the treated pigs consumed significantly less feed per day than the control pigs (7.03 lbs as compared to 8.40 lbs). In addition, the treated pigs displayed a marked improvement in carcass quality; the carcasses of the porcine ST treated pigs averaged 30.57 inches in length and had 1.40 inches of backfat, while those of the control group averaged 29.33 inches in length and had 1.77 inches of backfat. The chemical composition of the edible meat was also markedly improved in the porcine ST treated animals; 13.50% protein, 49.14% moisture and 36.76% fat, as compared to the control group's 10.8% protein, 39.43% moisture and 49.27% fat, e.g. E.J. Truman, "Some Effects of Pituitary Anterior Growth Hormone On Swine", Thesis; Purdue University (April 1953).

ST can be isolated from excised pituitary tissue. See, e.g., C.H. Li, J. Biol. Chem. 211, 55 (1954). ST can also be obtained from genetically engineered microorganisms containing recombinant DNA which specifies the production of ST. See, e.g., P.H. Seeburg, et al., Nature, 276, 795-798 (1978); P.H. Seeburg et al., Nature, 270, 486-494 (1978); J.A. Martial, Science, 205, 602-607 (1979).

Bovine Somatotropin (bST), also referred to in the art as Bovine Growth Hormone (bGH), is a polypeptide synthesized in and secreted from the anterior lobe of the pituitary. bST is believed to be synthesized as a precursor protein and to be matured to bST during secretion and release into the blood stream. Moreover, it has been reported that protein fractions of natural bST include polypeptides that lack a number of amino-terminal amino acids, but which are still biologically active.

A nucleotide coding sequence and an amino acid sequence of native bST have been reported; e.g. W.L. Miller et al., J. Biol. Chem., 255, 7521-24 (1980); M. D. Dayhoff et al., in "Atlas of Protein Sequence And Structure", Dayhoff ed., 5, Supp. 3, 345-42 (1978); and M. Wallis, FEBS Lett, 35, 11-14 (1973). bST is a polypeptide of 191 amino acids and appears to be synthesized initially as a bovine pre-somatotropin of 217 amino acids; the signal sequence of 26 amino acids is removed from the N-terminal position during synthesis and secretion, e.g. V.R. Lingapa et al., Proc. Natl. Acad. Sci. USA, 74, 2432-36 (1977).

The preparation of bST is well known in the art. For example, bST is extracted from pituitary glands of cattle or produced via recombinant DNA technology in appropriate hosts, e.g. W.L. Miller et al., J. Biol. Chem., 255, 7521-24 (1980). U.S. Patent No. 4,443,539 to Frazier et al, incorporated by reference herein, discloses a process for preparing bST by utilizing recombinant DNA methodology to place the bST structural gene into yeast cells. U.S. Patent No. 4,371,462 to Hecht, incorporated by reference herein, discloses a method for the purification of anterior pituitary peptides. European Patent Application Nos. 83304574.3, filed August 8, 1983, with Publication Number 103,395; 82304880.6, filed September 16, 1982, with Publication Number 075,444; and 81303824.7, filed August 21, 1981, with Publication Number 047,600; and British Patent Application No. 2,073,245A disclose methods for producing recombinant bST in high yields. Strains of E. Coli that produce bST are available from the American Type Culture Collection under accession numbers ATCC 31826, 31840, 31841, 31842, and 31843.

ST produced in animals of different species vary in antigens induced, isoelectric points, N-terminal and C-terminal amino acid residues, and amino acid composition. ST is generally species-specific, that is, ST from one species is inactive in another species. However, a ST from one species may be biologically active in another species lower in the evolutionary scale, although such trans-species activities are highly unpredictable.

This species specific limitation on the use of ST has several disadvantages. Equipment, expertise, recombinant microorganism strains, process and handling conditions, etc. must be obtained or developed for the production of ST for each desired species. Additionally, ST for one species may be more costly to produce, more difficult to recover, or less stable than ST for a different species. It would, therefore, save considerable expense and duplication of effort if, contrary to previous teachings, the ST from one species could be used to treat other non-related species. For example, a particular advantage in costs and duplication of effort could be saved if ST from one species could be used to promote growth in another species.

## Summary of the Invention

It is, therefore, an object of the present invention to provide a method for promoting growth in one species using somatotropin from another species.

It is another object of the present invention to provide a method for promoting growth in swine using bovine somatotropin.

It is another object of the present invention to provide a method for increasing weight gain in swine using bovine somatotropin.

It is a further object of the present invention to provide a method for increasing feed utilization efficiency in swine using bovine somatotropin.

These and other objects are achieved by administering bovine somatotropin (bST) to swine in amounts sufficient to promote growth, increase feed utilization efficiency, and improve weight gain.

In the preferred embodiment, bST is administered to swine in dosages from about 0.1-20 mg/animal/day, preferably 1-10 mg/animal/day, to promote growth, improve weight gain, and increase feed utilization efficiency.

Other objects, advantages, and novel features of the present invention will become apparent from the following detailed description of the invention.

## DETAILED DESCRIPTION OF THE INVENTION

According to the present invention, bovine somatotropin (bST) is administered to swine in amounts sufficient to promote growth, improve rate of weight gain, and increase feed utilization efficiency.

bST can be obtained from any suitable source. Methods for producing, isolating and purifying native and recombinant bST are well known in the field. bST as used herein includes all proteins having bST activity including natural, recombinant, and mutein proteins having deleted, inserted, replaced, or otherwise altered amino acid sequences. For example, delta-g bST, a polypeptide lacking the first nine amino-terminal amino acids of native ST, has been shown to retain biological activity. Production of a transformant strain of E. Coli HB101 containing the plasmids for encoding delta-9 bST is disclosed in European Patent Application Publication No. 0 103 395, incorporated herein by reference. Other such modified bST proteins retaining biological activity, well known in the art, are within the scope of the present invention.

Although the dosages of bST vary according to the age, size, and character of the particular swine, bST is typically administered to the animal in dosages from about 0.1-20 mg/animal/day, preferably from about 1-10 mg/animal/day.

bST according to the present invention can be administered to the swine in any acceptable manner including by injection, using an implant, and the like. Injections and implants are preferred because they permit precise control of the timing and dosage levels used for administration. bST according to the present invention is preferably administered parenterally. As used herein, parenteral administration means by intravenous, intramuscular, or intraperitoneal injection, or by subcutaneous implant.

When administered by injection, bST according to the present invention can be administered to the swine in an injectable formulation containing any biocompatible and bST compatible carrier such as various vehicles, adjuvants, additives, and diluents. bST according to the present invention is added to the carrier in amounts sufficient to supply from about 0.1-20 mg/animal to the swine when injected. Preferably, bST according to the present invention is added to a buffer containing about 0.025 M $NaHCO_3$ and about 0.025 M $Na_2CO_3$ in amounts sufficient to supply from about 1-10 mg/animal.

Aqueous vehicles, such as water having no nonvolatile pyrogens, sterile water, and bacteriostatic water, are also suitable to form injectable bST solutions. In addition to these forms of water, several other aqueous vehicles can be used. These include isotonic injection compositions that can be sterilized, such as sodium chloride, Ringer's, dextrose, dextrose and sodium chloride, and lactated Ringer's. Addition of water-miscible solvents, such as methanol, ethanol, or propylene glycol, generally increases solubility and stability of bST in these vehicles.

Nonaqueous vehicles, such as cottonseed oil, sesame oil, or peanut oil and esters such as isopropyl myristate, may also be used as solvent systems for bST compositions.

Additionally, various additives which enhance the stability, sterility, and isotonicity of the composition including antimicrobial preservatives, antioxidants, chelating agents, and buffers can be added. Any vehicle, diluent, or additive used would, however, have to be biocompatible and compatible with bST according to the present invention. Preferably bST is administered in a buffer containing about 0.025 M $NaHCO_3$ and about 0.025 M $Na_2CO_3$.

bST according to the present invention can be administered to the swine in the form of a slow-release subcutaneous implant which is inserted beneath the skin of the swine. The implant can take the form of a pellet which slowly dissolves after being implanted in the swine or a biocompatible and swine compatible delivery module well known to those skilled in the art. Such well known dosage forms are designed such that the active ingredients are slowly released over a period of several days to several weeks. The implant is designed to deliver from about 0.1-20 mg/animal/day, preferably from about 1-10 mg/animal/day.

According to the present invention, a composition for promoting growth, improving weight gain, and increasing feed utilization efficiency in swine comprises a pharmaceutically acceptable carrier and a growth promoting, weight gain improving, and feed utilization efficiency increasing amount of bovine somatotropin (bST) admixed with the carrier. bST as used herein includes all proteins having bST activity including natural, recombinant, and mutein proteins having deleted, inserted, replaced, or otherwise altered amino acid sequences, including delta-9 bST and similar proteins having biological activity. The composition of the present invention contains the compounds in amounts sufficient to supply from about 0.1-20 mg/animal/day, preferably from about 1-10 mg/animal/day.

The composition can be in a form suitable for parenteral administration, typically suspensions, solutions, emulsions, injectable formulations, implants, and the like.

The compounds according to the present invention can be administered to the animals in a composition comprising an implant or injectable formulation containing any biocompatible and compound compatible carrier such as various vehicles, adjuvants, additives, and diluents.

Preferably, the composition according to the present invention is (1) an implant pellet comprising a biocompatible and bST compatible implant material and a growth promoting, weight gain improving, and feed utilization efficiency increasing amount of bovine somatotropin or (2) an injectable formulation comprising a biocompatible and bST compatible carrier and a growth promoting, weight gain improving, and feed utilization efficiency increasing amount of bovine somatotropin

It is especially advantageous to formulate the compounds of the present invention in unit dosage form for ease of administration and uniformity of dosage. Unit dosage form as used herein refers to physically discrete units suited as unitary dosages for the subject to be treated. Each unit contains a predetermined quantity of the compound calculated to produce the desired therapeutic effect in association with the pharmaceutically acceptable carrier. The specific unit dosage form is dictated by and directly dependent upon (a) the unique characteristics of the particular composition and (b) the particular therapeutic or prophylactic effect to be achieved.

bST according to the present invention is used to promote growth, improve weight gain, and increase feed utilization efficiency in swine.

The invention having been generally described, the following examples are given as particular embodiments of the invention and to demonstrate the practice and advantages thereof. It is understood that the examples are given by way of illustration and are not intended to limit the specification or the claims to follow in any manner.

## IN VIVO EXPERIMENTAL PROCEDURE

### EXAMPLE I

Crossbred barrows (20 kg) were purchased locally and acclimated to ration (Table I) and facility. Two weeks of background information from individual pens was obtained prior to starting the test. Thirty-four animals were selected from the original group based upon performance during acclimation. Treatments were randomly assigned to animals such that there were two groups of five animals each (treatments 4A and 4B), and three groups of eight animals each (treatments 1-3) as follows:

Treatment 1: Untreated control
Treatment 2: rpST, 3 mg/hd/day
Treatment 3: nbST, 3 mg/hd/day
Treatment 4A: rbST, 3 mg/hd/day (lot #JES-1)
Treatment 4B: rbST, 3 mg/hd/day (lot #PP#1)

Purified recombinant derived rpST (lot # Tris-01-00A.94.1) and rbST (lot #JES-1 and PP#1) were tested for purity and homogeneity prior to use in the experiment. Purified pituitary derived bST (lot #AFP-8414C) was purchased from an outside source and also tested for purity and homogeneity.

The animals were given feed (Table 1) and water ad libitum. Feed consumption data was collected at five day intervals between the hours of 9:00 and 10:00 a.m. The test period during which the barrows received treatment was 20 days in length.

Injectable solutions of ST were prepared using a buffer containing 0.025 M $NaHCO_3$ and 0.025 M $Na_2CO_3$ (Peel et al., Effect of Exogeneous Growth Hormone on Lactational Performance in High Yielding Cows, J. Nutr. 11:1662 (1981)). The buffer vehicle was sterilized by filtration and aseptically added to the ST. Injection solutions were prepared on day 0 and day 10 of the 20 day test. The injection solutions were prepared and allocated into ten storage vials. Each vial contained enough ST for one test day and was frozen until use. Upon use, the solution (enough for one day) was thawed in the refrigerator overnight where it remained until used for injection. Daily intramuscular injections, given between 9:00 and 10:00 a.m. were administered in the dorsal neck region of each animal. Injection volume was 3 ml of 1.0 mg/ml ST per head daily. The injection site (right or left side of the neck) was alternated between days. A new sterile injection needle (1 inch in length; gauge 21) was used for each pig.

The condition of the treatment site and general condition of the animals was monitored routinely by the veterinarian in charge.

Feed samples were collected and analyzed for percent protein, fat, fiber, calcium and phosphorous on each batch prepared. No contaminants in either the feed or the water were known which may have contributed to or interfered with the test results.

Production measurements taken were: average daily weight gain (ADG), feed per gain (F/G), and average daily feed intake (ADF). Serum data included: somatotropin (ST) and blood urea nitrogen (BUN). BUN was determined by the procedures of Tiffany et al., Enzymatic Kinetic Rate and End-Point Analysis of Substrate by use of GeMSAEC Fast Analyzer, Clin. Chem. 18:829 (1972). Serum ST assays were performed using procedures well known in the art.

The experiment was conducted as a randomized block design. Treatment groups consisted of eight replicates except in the case of rbST treatments where there were five replicates. Individual animals served as a replicate and were grouped by location. Pen location (north or south side of building) and ST treatment served as the two trial factors. Pigs were randomly assigned to pens and, within location, pigs were randomly allotted to one of five ST treatments.

For the performance data, extreme values were detected by the outlier test suggested by Gill, Design and Analysis of Experiments in the Animal and Medical Sciences. Volume I & II, The Iowa State University Press, Ames (1978). Extreme values consisted chiefly of occasional animals which lost weight or animals which habitually wasted feed. No treatment related incidences of poor performance or behavioral changes were noted.

Statistical analyses were performed utilizing well known analysis of variance and regression techniques BBN, RS/1 User's Guide 1983. BBN Research Systems, Bolt Beranek and Newman Inc., Cambridge, Ma. Dixon, W.J. (Ed) 1981. BMDP Statistical Software. University of California Press, Los Angeles.

nbST and rbST. Since statistical analysis revealed no significant interaction $(P > .10)$ of treatment effect by location (north or south side of building), only the main effect ST treatment results are presented. Table 2 shows the mean performance data.

Referring to Table 2, both rpST and nbST improved feed conversion $(P < .01)$ when compared to untreated control animals. After twenty days, rpST and nbST had improved feed conversion efficiencies 29% and 27%, respectively. A 17% improvement $(P < .05)$ in F/G was also evident for rbST (lot #JES-1) after twenty days, but lot PP-1 of rbST did not significantly $(P > .05)$ affect F/G.

Referring again to Table 2, rPST also improved ADG $(P < .05)$. No statistically significant differences $(P > .05)$ in gain or intake could be determined when comparing either lot of rbST to untreated controls.

No detrimental effects from ST or delivery system were observed on animal health or performance throughout the study and post-treatment observation period.

Obviously many modifications and variations of the present invention are possible in light of the above teachings. It is therefore to be understood that within the scope of the appended claims the invention may be practiced otherwise than as specifically described.

## Table 1

### Ration Composition

| Item | Percent | Actual* |
|------|---------|---------|
| Ground yellow corn | 73.5 | |
| Soybean meal - 44% | 23.5 | |
| Dicalcium phosphate | 1.2 | |
| Limestone | 0.8 | |
| Salt | 0.5 | |
| Dawes vitamin/mineral premix | 0.5 | |
| Calculated composition (as-fed basis) | | |
|     Crude protein % | 16.8 | 16.4 |
|     Calcium % | 0.62 | 0.70 |
|     Phosphorus % | 0.54 | 0.54 |

* Mean assay results for six lots of feed

## Effect of Various Somatotropin Types on Pig Performance

| Item | Control Neg. | rpST | nbST | rbST JES-1 | rbST PP-1 |
|---|---|---|---|---|---|
| **Days -1 to 5** | | | | | |
| No. Animals | 8 | 8 | 8 | 5 | 5 |
| ADG, kg | 0.87 | 1.19* | 1.15 | 1.01 | 1.16 |
| ADF, kg | 3.53 | 3.38 | 2.95 | 3.33 | 3.85 |
| F/G | 4.37 | 2.88** | 2.67** | 3.30 | 3.73 |
| **Days 6 to 10** | | | | | |
| No. Animals | 8 | 8 | 8 | 5 | 5 |
| ADG, kg | 0.96 | 0.97 | 0.95 | 0.94 | 0.84 |
| ADF, kg | 3.52 | 3.05 | 2.75* | 3.09 | 3.28 |
| F/G | 3.88 | 3.27 | 2.91 | 3.40 | 3.95 |
| **Days 11 to 15** | | | | | |
| No. Animals | 7 | 8 | 7 | 4 | 4 |
| ADG, kg | 0.64 | 0.77 | 0.63 | 0.76 | 0.82 |
| ADF, kg | 3.32 | 2.91 | 2.71* | 2.96 | 3.14 |
| F/G | 5.74 | 3.84 | 5.01 | 4.15 | 3.91 |
| **Days 16 to 20** | | | | | |
| No. Animals | 7 | 7 | 8 | 5 | 5 |
| ADG, kg | 0.92 | 1.17 | 0.82 | 0.87 | 0.97 |
| ADF, kg | 3.88 | 3.18 | 2.71* | 3.17 | 3.56 |
| F/G | 4.37 | 2.77** | 3.41 | 3.75 | 3.70 |
| **Days -1 to 20** | | | | | |
| No. Animals | 7 | 7 | 8 | 5 | 5 |
| ADG, kg[b] | 0.85 | 1.03* | 0.87 | 0.87 | 0.92 |
| ADF, kg | 3.66 | 3.15 | 2.73** | 3.12 | 3.47 |
| F/G | 4.35 | 3.08** | 3.17** | 3.59* | 3.76 |

* Differs from the mean of the negative control (P<.05)
** Differs from the mean of the negative control (P<.01)

## Claims

1. A method for promoting growth, improving weight gain, and increasing feed utilization efficiency in swine, comprising:

administering a growth promoting, weight gain improving, and feed utilization efficiency increasing amount of bovine somatotropin to said swine.

2. The method of Claim 1 wherein said bovine somatotropin is administered in amounts of from about 0.1-20 mg/animal/day.

3. The method of Claim 1 or Claim 2 wherein said bovine somatotropin is administered in amounts of from about 1-10 mg/animal/day.

4. The method of any one of Claims 1 to 3 wherein said bovine somatotropin is administered parenterally.

5. The method of any one of Claims 1 to 4 wherein said bovine somatotropin is administered using an implant, said implant further comprising:

a biocompatible and bovine somatotropin compatible implant material; and

a growth promoting, weight gain improving, and feed utilization efficiency increasing amount of said bovine somatotropin.

6. The method of any one of Claims 1 to 3 where said bovine somatotropin is administered in an injectable formulation, said injectable formulation further comprising:

a biocompatible and bovine somatotropin compatible carrier; and

a growth promoting, weight gain improving, and feed utilization efficiency increasing amount of said bovine somatotropin.

7. The method of Claim 6 wherein said carrier is a buffer containing about 0.025 M $NaHCO_3$ and about 0.025 M $Na_2CO_3$.

8. The method of any one of Claims 1 to 7 wherein said bovine somatotropin is a recombinant bovine somatotropin.

9. The method of Claim 8 wherein said recombinant bovine somatotropin has deleted, inserted, replaced, or otherwise altered amino acid sequences.

10. The method of Claim 8 or Claim 9 wherein said recombinant bovine somatotropin is delta-9 bovine somatotropin.

11. A composition for promoting growth, improving weight gain, and increasing feed utilization efficiency in swine, comprising:

a pharmaceutically acceptable carrier; and

a growth promoting, weight gain improving, and feed utilization efficiency increasing amount of bovine somatotropin.

12. The composition of Claim 10 containing said bovine somatotropin in amounts sufficient to supply from about 0.1-20 mg/animal/day.

13. The composition of Claim 10 or Claim 11 containing said bovine somatotropin in amounts sufficient to supply from about 1-10 mg/animal/day.

14. The composition of any one of Claims 10 to 12 in a form suitable for parenteral administration.

15. The composition of any one of Claims 10 to 12 in the form of an implant, said implant further comprising:

a biocompatible and bovine somatotropin compatible implant material; and

a growth promoting, weight gain improving, and feed utilization efficiency increasing amount of said bovine somatotropin.

16. The composition of any one of Claims 10 to 13 in the form of an injectable formulation, said injectable formulation further comprising:

a biocompatible and bovine somatotropin compatible carrier; and

a growth promoting, weight gain improving, and feed utilization efficiency increasing amount of said bovine somatotropin.

17. The composition of any one of Claims 10 to 15 wherein said bovine somatotropin is a recombinant bovine somatotropin.

18. The composition of Claim 16 wherein said recombinant bovine somatotropin has deleted, inserted, replaced, or otherwise altered amino acid sequences.

19 The composition of Claim 16, or Claim 17 wherein said recombinant bovine somatotropin is delta-9 bovine somatotropin.

20. Use of bovine somatotropin for preparation of a composition for promotion of growth in swine.